# FASCICULE DE BREVET EUROPEEN

(11) **EP 1 814 901 B1**
(45) Date de publication et mention de la délivrance du brevet: **16.07.2008**
(21) Numéro de dépôt: 05815291.9
(22) Date de dépôt: 03.11.2005
(51) Int. Cl.: C07K 7/06, C07K 7/08, G01N 33/53, A61K 39/00

(54) **PEPTIDES CITRULLINES DERIVES DE LA FIBRINE RECONNUS PAR DES AUTO-ANTICORPS SPECIFIQUES DE LA POLYARTRHITE RHUMATOIDE, ET LEURS UTILISATIONS**
VON FIBRIN ABGELEITETE CITRULLINPEPTIDE, DIE DURCH FÜR RHEUMATOIDE ARTHRITIS SPEZIFISCHE AUTOANTIKÖRPER ERKANNT WERDEN, UND DEREN VERWENDUNG
CITRULLINE PEPTIDES DERIVED FROM FIBRIN AND RECOGNISED BY RHEUMATOID ARTHRITIS SPECIFIC AUTOANTIBODIES AND THE USE THEREOF

(30) Priorité: 04.11.2004 FR 0411782; 22.12.2004 FR 0413711; 08.08.2005 FR 0508422
(43) Date de publication de la demande: 08.08.2007
(73) Titulaire: BIOMERIEUX, 69280 Marcy L'Etoile (FR)
(72) Inventeur: SERRE, Guy, Résidence du Lac-Appartement 46, 31100 TOULOUSE (FR); SEBBAG, Mireille, F-31400 TOULOUSE (FR)
(74) Mandataire: Vialle-Presles, Marie José
(86) Numéro de dépôt international: PCT/FR2005/002736
(87) Numéro de publication internationale: WO 2006/048556

(56) Documents cités:
- WO-A-01/02437
- WO-A-03/050542
- SEBBAG M ET AL: "Clinical and pathophysiological significance of the autoimmune response to citrullinated proteins in rheumatoid arthritis" JOINT BONE SPINE, ELSEVIER, PARIS, FR, vol. 71, no. 6, novembre 2004 (2004-11), pages 493-502, XP004678687 ISSN: 1297-319X
- NIJENHUIS S ET AL: "Autoantibodies to citrullinated proteins in rheumatoid arthritis: clinical performance and biochemical aspects of an RA-specific marker" CLINICA CHIMICA ACTA, AMSTERDAM, NL, vol. 350, no. 1-2, décembre 2004 (2004-12), pages 17-34, XP004629806 ISSN: 0009-8981
- HIDA S ET AL: "Influence of arginine deimination on antigenicity of fibrinogen" JOURNAL OF AUTOIMMUNITY, LONDON, GB, vol. 23, no. 2, septembre 2004 (2004-09), pages 141-150, XP004590158 ISSN: 0896-8411
- SCHELLEKENS G A ET AL: "THE DIAGNOSTIC PROPERTIES OF RHEUMATOID ARTHRITIS ANTIBODIES RECOGNIZING A CYCLIC CITRULLINATED PEPTIDE" ARTHRITIS AND RHEUMATISM, LIPPINCOTT, PHILADELPHIA, US, vol. 43, no. 1, janvier 2000 (2000-01), pages 155-163, XP008018548 ISSN: 0004-3591

## Description

La présente Invention est relative à de nouveaux peptides citrullinés reconnus par des auto-anticorps spécifiques de la polyarthrite rhumatoïde.

La polyarthrite rhumatoïde (ci après abrégée en "PR ") est le plus fréquent des rhumatismes inflammatoires chroniques. Il s'agit d'une maladie auto-immune, et le sérum des patients atteints contient des auto-anticorps dont certains sont spécifiques, et peuvent constituer un marqueur de cette maladie, permettant son diagnostic même à des stades précoces. Des recherches ont donc été effectuées en vue d'identifier des antigènes reconnus par ces anticorps, afin d'en obtenir des préparations purifiées utilisables dans des techniques classiques de diagnostic immunologique.

Il a été montré que des auto-anticorps spécifiques de la PR reconnaissaient différents variants isoélectriques de la (pro)filaggrine (pour revue, cf. par exemple SERRE et VINCENT, In : Autoantibodies, PETER and SHOENFELD Eds, Elsevier Science Publishers, 271-276, 1996). Ces auto-anticorps ont pour cette raison été dénommés : « auto-anticorps anti-filaggrine (AAF) ». La Demande EP 0 511 116 décrit la purification et la caractérisation d'antigènes de la famille des filaggrines reconnus par ces anticorps, et leur utilisation pour le diagnostic de la polyarthrite rhumatoïde.

Ultérieurement, les épitopes de la filaggrine reconnus par les AAF ont été identifiés comme des régions de la molécule de filaggrine porteuses de résidus citrullyl, résultant de la transformation des résidus arginyl par une peptidylarginine désiminase (Girbal-Neuhauser E et al., J Immunol, 162, 585-94, 1999 ; Schellekens GA et al., J Clin Invest, 101, 273-81, 1998). L'analyse de divers peptides synthétiques dérivés de la séquence de la filaggrine humaine a montré que la désimination (citrullination) était nécessaire à la constitution des épitopes reconnus par les AAF, qui sont aujourd'hui également appelés auto-anticorps anti-protéines citrullinées (AAPC). C'est cette dénomination qui sera utilisée dans l'exposé qui va suivre.

Il a également été observé que l'environnement des résidus citrullyl jouait aussi un rôle important (Girbal-Neuhauser E, 1999, précité; Schellekens GA, 1998, précité; Union A et al., Arthritis Rheum, 46, 1185-95, 2002) ; certains acides aminés « permissifs » vis-à-vis de la fixation de l'anticorps et dont la nature module probablement l'affinité de fixation de l'anticorps ont été identifiés. Parmi ces acides aminés, les résidus -gly-, -ser-, -his-, -thr- et -gln-, sont les plus souvent retrouvés au niveau de l'environnement immédiat des résidus citrullyl des peptides "filaggrine" citrullinés qui, à ce jour, ont été identifiés comme porteurs d'épitopes reconnus par les AAPC (Sebbag M et al., Rev Rhum, 68, 106, 2001).

De très nombreux peptides citrullinés spécifiquement reconnus par des AAPC, et utilisables pour le diagnostic de la PR ont été obtenus à partir de la filaggrine. Cependant, il a également été observé que bien qu'étroitement spécifique de la PR, la réactivité de ces différents peptides citrullinés vis-à-vis des AAPC était hétérogène, des peptides différents étant reconnus par des sérums issus de sujets différents. Ceci implique que pour obtenir un réactif de diagnostic capable d'identifier la présence d'AAPC dans une large population, il est nécessaire d'associer plusieurs peptides différents.

Parallèlement, il a été montré que les AAPC sont sécrétés par les plasmocytes du tissu synovial (Masson-Bessière C et al., Clin Exp Immunol, 119, 544-52, 2000) et sont spécifiquement dirigés contre des formes citrullinées des chaînes α et β de fibrine présentes dans ce tissu (Masson-Bessière C et al., J Immunol, 166, 4177-84, 2001).

WO 01/02437 et Sebbag et al. (Joint Bone Spine vol.71, 2004, p.493-502) divulguent l'utilisation de dérivés citrullinés de la fibrine pour le diagnostic de la polyarthrite rhumatoïde. Nijenhuis et al. (Clinica Chimica Acta vol.350, 2004, p.17-34) discutent les avantages de peptides citrullinés par rapport aux protéines citrullinées pour le diagnostic de la polyarthrite rhumatoïde.

Les Inventeurs ont entrepris, dans le but de mieux caractériser les épitopes reconnus par les AAPC, d'identifier ceux présentés par les chaînes α et β de la fibrine. Dans ce but, ils ont évalué la réactivité de sérums contenant des AAPC vis-à-vis de peptides synthétiques citrullinés dérivés de la séquence de la chaîne α et de la séquence de la chaîne β de la fibrine. Compte tenu de l'hétérogénéité connue des profils réactionnels des peptides citrullinés issus de la filaggrine avec les AAPC, les Inventeurs ont utilisé des mélanges de sérums choisis de manière à contenir des AAPC représentant les différents profils de réactivité observés dans le cas de ces peptides issus de la filaggrine, afin de détecter tous les peptides de la fibrine pouvant être reconnus par des AAPC.

Les peptides testés ont été obtenus à partir de la séquence de la chaîne α et de la séquence de la chaîne β de la fibrine. Au total, 72 peptides, dont 41 dérivés de la chaîne α de la fibrine et 31 dérivés de sa chaîne β ont été choisis. Parmi les 72 peptides citrullinés analysés, les Inventeurs ont identifié 13 peptides dérivés de la séquence de la chaîne α et 5 peptides dérivés de celle de la chaîne β de la fibrine comme réagissant significativement avec l'un et/ou l'autre des mélanges de sérums testés, et donc comme étant porteurs d'épitopes reconnus par certains des AAPC présents dans ce(s) mélange(s).

Les Inventeurs ont ensuite analysé individuellement chacun des 18 peptides réactifs identifiés avec chacun des sérums constitutifs des mélanges testés. Cette analyse a confirmé, pour la majeure partie des peptides testés, la large variabilité interindividuelle de spécificité des AAPC précédemment observée dans le cas des peptides citrullinés dérivés de la filaggrine.

D'autre part, elle a permis aux Inventeurs d'identifier des peptides qui, de manière inattendue, possèdent un spectre de réactivité avec les AAPC beaucoup plus large que ceux rapportés jusqu'à présent dans le cas des peptides citrullinés dérivés de la filaggrine. En effet, les Inventeurs ont identifié 5 peptides citrullinés (4 peptides dérivés de la chaîne α et 1 dérivé de la chaîne β de la fibrine), qui sont chacun reconnus individuellement par au moins 40% des sérums analysés, et apparaissent donc porteurs d'épitopes majeurs. Parmi ces peptides, deux sont reconnus par la majorité des sérums, et présentent en outre des profils de réactivité complémentaires englobant la totalité des sérums analysés. Chacun de ces sérums reconnaît en effet l'un et/ou l'autre de ces peptides. Ceci suggère que ces deux peptides sont porteurs de motifs structuraux représentatifs d'une très large majorité des différents motifs reconnus par les AAPC.

La présente invention a pour objet un peptide isolé reconnu par des auto-anticorps anti-protéines citrullinées (AAPC) présents dans le sérum des patients atteints de polyarthrite rhumatoïde, caractérisé en ce qu'il comprend au moins un résidu citrullyl, et en ce qu'il est choisi dans le groupe constitué par :
a) un peptide défini par la séquence X₁PAPPPISGGGYX₂AX₃ (SEQ ID NO: 1) dans laquelle X₁, X₂, et X₃ représentent chacun un résidu citrullyl ou un résidu arginyl, et au moins l'un des résidus X₂ ou X₃ est un résidu citrullyl ;
b) un peptide défini par la séquence GPX₁VVEX₂HQSACKDS (SEQ ID NO: 2) dans laquelle X₁, et X₂ représentent chacun un résidu citrullyl ou un résidu arginyl, et au moins l'un des résidus X₁ ou X₂ est un résidu citrullyl ;
c) un peptide défini par la séquence SGIGTLDGFX₁HX₂HPD (SEQ ID NO: 3) dans laquelle X₁, et X₂ représentent chacun un résidu citrullyl ou un résidu arginyl, et au moins l'un des résidus X₁ ou X₂ est un résidu citrullyl ;
d) un peptide défini par la séquence VDIDIKIX₁SCX₂GSCS (SEQ ID NO: 4) dans laquelle X₁ et X₂ représentent chacun un résidu citrullyl ou un résidu arginyl, et au moins l'un des résidus X₁ ou X₂ est un résidu citrullyl ;
e) un peptide défini par la séquence X₁GHAKSX₂PVX₃GIHTS (SEQ ID NO: 12) dans laquelle X₁, X₂ et X₃ représentent chacun un résidu citrullyl ou un résidu arginyl, et au moins l'un des résidus X₁, X₂ ou X₃ est un résidu citrullyl ;
f) un peptide comprenant au moins 5 acides aminés consécutifs, de préférence au moins 7 acides aminés consécutifs, et avantageusement, de 8 à 14 acides aminés consécutifs, dont au moins un résidu citrullyl, de l'un des peptides a) à e) ci-dessus.

Les peptides conformes à l'invention ont une taille d'au moins 5 acides aminés, de préférence une taille de 5 à 25 acides aminés, et, de manière tout à fait préférée, une taille de 10 à 20 acides aminés.

Selon un mode de réalisation préféré d'un peptide conforme à l'invention il est choisi parmi :
- un peptide défini par la séquence SEQ ID NO: 1 dans laquelle au moins X₃ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 2 dans laquelle au moins X₂ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 3 dans laquelle au moins X₂ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 4 dans laquelle au moins X₁ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 12 dans laquelle au moins X₃ est un résidu citrullyl, ou un peptide comprenant un fragment d'au moins 5 acides aminés consécutifs de ladite séquence contenant ledit résidu citrullyl.

De manière particulièrement préférée, un peptide conforme à l'invention est choisi parmi :
- un peptide défini par la séquence SEQ ID NO: 1 dans laquelle X₁, X₂, et X₃ sont des résidus citrullyl, ou un peptide d'au moins 16 acides aminés comprenant ladite séquence ;
- un peptide défini par la séquence SEQ ID NO: 2 dans laquelle X₁ et X₂ sont des résidus citrullyl, ou un peptide comprenant un fragment d'au moins 5 acides aminés consécutifs de ladite séquence contenant lesdits résidus citrullyl ;
- un peptide défini par la séquence SEQ ID NO: 3 dans laquelle X₁ et X₂ sont des résidus citrullyl, ou un peptide comprenant un fragment d'au moins 5 acides aminés consécutifs de ladite séquence contenant lesdits résidus citrullyl ;
- un peptide défini par la séquence SEQ ID NO: 4 dans laquelle X₁ et X₂ sont des résidus citrullyl, ou un peptide comprenant un fragment d'au moins 5 acides aminés consécutifs de ladite séquence contenant lesdits résidus citrullyl ;
- un peptide défini par la séquence SEQ ID NO: 12 dans laquelle X₁, X₂ et X₃ sont des résidus citrullyl, ou un peptide comprenant un fragment d'au moins 10 acides aminés consécutifs de ladite séquence contenant lesdits résidus citrullyl.

Des peptides citrullinés conformes à l'invention peuvent par exemple être obtenus à partir de fragments de fibrine ou de fibrinogène naturels, recombinants, ou de synthèse, par l'action de la peptidyl arginine déiminase (PAD) ; ils peuvent également être obtenus par synthèse peptidique, en incorporant directement un ou plusieurs résidus citrulline, dans le peptide synthétisé.

Des peptides citrullinés conformes à l'invention englobent également des dérivés des peptides SEQ ID NO: 1 à 4, et 12 ou des fragments de ceux-ci, définis ci-dessus, lesdits dérivés portant des modifications destinées à améliorer leur reconnaissance par les AAPC : à titre d'exemples de tels dérivés, on citera : des peptides cyclisés ; des peptides de type rétro, dans lesquels des acides L-aminés sont enchaînés selon une séquence inverse de celle du peptide à reproduire ; des peptides de type *rétro-inverso,* constitués par des acides aminés de la série D (au lieu des acides aminés de la série L des peptides naturels) enchaînés selon une séquence inverse de celle du peptide à reproduire.

Très avantageusement, il s'agit de peptides dans lesquels la fonction carboxyle (COOH) terminale est remplacée par une fonction carboxamide (CONH₂). Dans ce cadre, des peptides particulièrement préférés sont ceux dans lesquels le résidu C-terminal est un résidu citrullyl dont la fonction carboxyle est remplacée par une fonction carboxamide.

La présente invention a également pour objet tout peptide reconnu par des AAPC présents dans le sérum des patients atteints de polyarthrite rhumatoïde, et contenant à son extrémité C-terminale un résidu citrullyl dont la fonction carboxyle est remplacée par une fonction carboxamide. Avantageusement, ledit peptide contient dans sa séquence au moins un autre résidu citrullyl. De préférence, ledit peptide comprend de 5 à 25 acides aminés.

Le remplacement de la fonction carboxyle du résidu citrulline C-terminal par une fonction carboxamide peut permettre d'augmenter la réactivité du peptide avec les AAPC, ou, le cas échéant, de rendre réactifs avec des AAPC, des peptides qui ne le sont pas naturellement.

Des peptides citrullinés conformes à l'invention englobent également des dérivés des peptides SEQ ID NO: 1 à 4, et 12 ou des fragments de ceux-ci, tels que définis ci-dessus, lesdits dérivés portant des modifications destinées à faciliter leur synthèse et/ou à améliorer leur stabilité. A titre d'exemple de tels dérivés, on citera les peptides incluant des acides aminés dont les groupements carboxyl sont estérifiés ou transformés en groupements amide et/ou des acides aminés dont un groupement aminé est alkylé, par exemple méthylé ou acétylé. Les groupements amine et carboxyl des peptides peuvent être présents sous forme du sel correspondant à la base ou à l'acide.

A partir des peptides citrullinés décrits ci-dessus, il est aussi possible d'obtenir des peptides mimotopes comprenant au moins un résidu citrullyle (peptide mimotope citrulliné).

Ces peptides mimotopes peuvent être obtenus en criblant des banques de peptides citrullinés dont les séquences sont définies à partir de celles des peptides SEQ ID n° 1, 2, 3, 4, 12 de la présente invention, utilisés dans ce cadre comme "peptides modèles".

De préférence, ces banques de peptides sont réalisées par synthèse de différents peptides de taille comprise entre 10 et 20, de préférence entre 12 et 17 acides aminés, en particulier 15 acides aminés. Chacun de ces peptides conserve au moins 2, de préférence au moins 4, avantageusement au moins 6, de manière particulièrement préférée au moins 8, et très avantageusement au moins 10 acides aminés, dont au moins un résidu citrullyle, de la séquence du peptide modèle choisi, aux mêmes positions que sur ledit peptide modèle, les autres positions étant variables.

Ces banques peuvent avantageusement être criblées comme décrit dans les exemples ci-après, et notamment à l'aide de sérums représentatifs de différents profils de réactivité des AAPC, tels que définis dans l'Exemple 1 ci-après.

Des méthodes de synthèse de peptides mimotopes sont bien connues en elles-mêmes. On se référera par exemple au chapitre 6 de "Chemical approaches to the synthesis of peptides and proteins", Paul Lloyd-Williams, Fernando Albericio and Ernest Giralt, CRC Press New York, 1997, "Peptides libraries", pages 237-270.

Ces peptides mimotopes citrullinés peuvent être utilisés, seuls ou en combinaison avec d'autres peptides citrullinés et de préférence en combinaison avec au moins l'un des autres peptides de l'invention, dans un test de diagnostic de la PR pour reconnaître les AAPC."

La présente Invention a également pour objet l'utilisation des peptides conformes à l'invention, tels que définis ci-dessus, pour détecter la présence d'AAPC dans un échantillon biologique, dans le cadre du diagnostic *in vitro* de la PR.

La présente invention a ainsi pour objet des compositions antigéniques, utilisables pour détecter la présence d'AAPC dans un échantillon biologique dans le cadre du diagnostic *in vitro* de la PR, lesquelles compositions sont caractérisées en ce qu'elles comprennent au moins un peptide conforme à l'invention.

Des compositions conformes à l'invention peuvent associer entre eux différents peptides choisis parmi les peptides conformes à l'invention, ou bien peuvent associer un ou plusieurs peptides conformes à l'invention avec un ou plusieurs peptides citrullinés dérivés notamment de la filaggrine.

Selon un mode de réalisation préféré d'une composition antigénique conforme à l'invention, elle comprend au moins un peptide de séquence SEQ ID NO: 1, et au moins un peptide de séquence SEQ ID NO: 2, tels que définis ci-dessus.

Cette composition possède un très large spectre de réactivité, et peut permettre en outre de détecter la PR à un stade précoce.

Avantageusement, une composition conforme à l'invention peut comprendre en outre un peptide de séquence SEQ ID NO: 3 et/ou un peptide de séquence SEQ ID NO: 4 et/ou un peptide de séquence SEQ ID NO: 12, tels que définis ci-dessus.

Les compositions conformes à l'invention peuvent, le cas échéant, se présenter sous forme de compositions multi-peptidiques, dans lesquelles les peptides constitutifs sont associés entre eux ou à une molécule porteuse généralement par liaison covalente. A titre d'exemple, on citera les multi-peptides antigéniques (MAP pour « multiple antigen peptides » décrits notamment par TAM (Proc. Natl. Acad. Sci. U.S.A., 85, 5409-13, 1988).

La présente Invention à également pour objet un procédé de détection de la présence d'AAPC dans un échantillon biologique, dans le cadre du diagnostic in vitro de la PR, lequel procédé est caractérisé en ce qu'il comprend :
- la mise en contact dudit échantillon biologique avec au moins un peptide ou une composition antigénique conforme à l'Invention, tel que définis ci-dessus, dans des conditions permettant la formation d'un complexe antigène/anticorps avec les AAPC éventuellement présents dans ledit échantillon ;
- la détection, par tous moyens appropriés, du complexe antigène/anticorps éventuellement formé.

Ce procédé de détection peut être mis en oeuvre grâce à un nécessaire comprenant au moins un peptide ou une composition antigénique selon l'Invention, et, le cas échéant, des tampons et réactifs appropriés pour la constitution d'un milieu réactionnel permettant la formation d'un complexe antigène/anticorps, et/ou des moyens de détection dudit complexe antigène/anticorps.

Avantageusement, ledit nécessaire comprend un peptide ou une composition antigénique selon l'Invention, immobilisé sur un support solide. A titre d'exemples non-limitatifs de supports solides utilisables, on citera des plaques de microtitration, des cônes de l'appareil VIDAS^{®} (commercialisé par BIOMERIEUX), des billes, des microbilles ou microparticules, des bandelettes, etc....

Ledit nécessaire peut également comprendre des échantillons de référence, tels qu'un ou plusieurs sérum(s) négatif(s) et un ou plusieurs sérum(s) positif(s).

La présente invention a aussi pour objet un procédé de détection de la présence d'AAPC dans un échantillon biologique, dans le cadre du diagnostic *in vitro* de la PR, lequel procédé est caractérisé en ce qu'il comprend :
- la fourniture d'un premier peptide citrulliné capable d'entrer en compétition pour la liaison auxdits AAPC avec un peptide conforme à l'Invention, tel que défini ci-dessus ;
- la mise en contact dudit premier peptide avec ledit échantillon biologique, dans des conditions permettant la formation d'un complexe antigène/anticorps avec les AAPC éventuellement présents dans ledit échantillon ;
- la détection, par tous moyens appropriés, du complexe antigène/anticorps éventuellement formé.
   En particulier, le premier peptide citrulliné capable d'entrer en compétition avec un peptide conforme à l'invention est un peptide mimotope citrulliné pouvant être obtenu comme décrit ci-dessus.
   La présente invention sera mieux comprise à l'aide du complément de description qui va suivre, qui se réfère à des exemples illustrant l'identification des peptides conformes à l'invention et la mise en évidence de leur profil de réactivité vis-à-vis des AAPC.

### EXEMPLE 1 : MISE EN EVIDENCE DE L'HETEROGENEITE DE LA REACTION DES AAPC AVEC DES PEPTIDES CITRULLINES ISSUS DE LA FILAGGRINE, ET OBTENTION DE MELANGES DE SERUMS REPRESENTATIFS DES DIFFERENTS PROFILS DE REACTIVITE.

90 sérums, présentant des AAPC détectables à la fois par ELISA et par immunotransfert sur fibrinogène humain désiminé in vitro (Masson-Bessière C, 2001, précité ; Nogueira L et al., Arthritis Res, 4, 90, 2002) mais aussi par immunofluorescence indirecte sur cryocoupes d'oesophage de rat (Vincent C et al., Ann Rheum Dis, 48, 712-22, 1989) et par immunotransfert sur filaggrine épidermique humaine (Vincent C et al., J Rheumatol, 25, 838-46, 1998) ont été utilisés. La réactivité de ces 90 sérums multi-positifs a été analysée en ELISA à l'égard de 5 peptides citrullinés -différents dont la réactivité vis-à-vis des AAPC avait été précédemment établie. Ces peptides sont les suivants :
E12D ESSRDGSXHPRSHD (SEQ ID NO : 5)
T12E TGSSTGGXQGSHHE (SEQ ID NO : 6)
E12H EQSADSSXHSGSGH (SEQ ID NO : 7)
cfc6 SHQESTXGXSRGRSGRSGS (SEQ ID NO : 8)
cf48-65-4 TIHAHPGSXXGGRHGYHH (SEQ ID NO : 9)
(X désigne un résidu citrullyl)

Les peptides E12D, T12E, et E12H ont été décrits par Girbal-Neuhauser, E. et al. (1999) les peptides cfc6 et cf48-65-4 ont été décrits par Schellekens, G. et al. (1998).

L'analyse par ELISA a été effectuée selon le protocole décrit par Girbal-Neuhauser, E. et al. (1999)

Cette analyse a permis d'identifier 12 profils de réactivité à l'égard des 5 peptides.

Ces profils sont résumés dans le Tableau I ci-dessous.

**Tableau I**

| Profil | E12D | E12H | T12E | cfc6 | cf48-65-4 |
|---|---|---|---|---|---|
| 1 | + | + | + | | + |
| 2 | + | + | + | | |
| 3 | + | + | | + | |
| 4 | + | + | | | |
| 5 | + | | | | |
| 6 | + | | | | + |
| 7 | | + | | | |
| 8 | | + | + | | + |
| 9 | | | + | | |
| 10 | | | | + | + |
| 11 | | | | + | |
| 12 | | | | | + |

De manière à être le plus représentatif possible des différents profils de réactivité des AAPC, des mélanges, dénommés ci-après mélanges : « A » et « B » ont chacun été constitués par mélange à parts égales de 10 sérums représentant différents profils de réactivité sur peptides "filaggrine".

La composition de ces deux mélanges est indiquée dans le Tableau II ci-dessous.

**Tableau II**

| Sérum | Profil | Mélange |
|---|---|---|
| 97.0459 | 1 | A |
| 97.0388 | 3 | |
| 97.1436 | 4 | |
| 97.0169 | 6 | |
| 97.0530 | 7 | |
| 97.0311 | 8 | |
| 97.0506 | 9 | |
| 97.0468 | 10 | |
| 97.0796 | 11 | |
| 97.0907 | 12 | |
| 97.1715 | 1 | B |
| 97.0524 | 1 | |
| 97.0323 | 2 | |
| 97.0794 | 4 | |
| 95.0256 | 5 | |
| 97.1795 | 5 | |
| 97.1474 | 9 | |
| 97.0244 | 10 | |
| 97.1548 | 11 | |
| 97.1210 | 12 | |

A eux deux, les mélanges A et B sont donc représentatifs de l'hétérogénéité de spécificité des sérums AAPC+.

### EXEMPLE 2 : IDENTIFICATION DE PEPTIDES CITRULLINES DERIVES DES CHAINES α ET β DE LA FIBRINE, REAGISSANT AVEC LES AAPC.

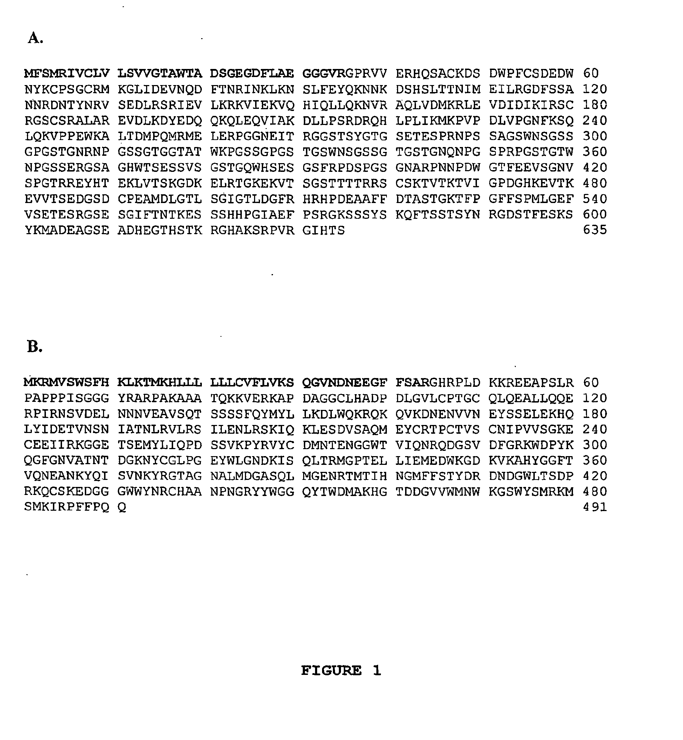

Les peptides testés ont été obtenus à partir de la séquence de la chaîne α et de la séquence de la chaîne β de la fibrine [portion correspondant respectivement aux résidus 36-635 et 45-491 des chaînes A(α) (référence NP Accession: NP_068657) et B(β) (référence SWISSPROT FIBB_HUMAN Prim. Accession: P02675) du fibrinogène]. Les séquences des résidus 1 à 635 et 1 à 491 des chaînes A(α) et B(β) du fibrinogène sont également respectivement représentées dans la liste de séquences en annexe sous les numéros SEQ ID NO: 10 et SEQ ID NO: 11, et sur la Figure 1 A et B. Les séquences indiquées en gras sur la Figure 1 sont celles des séquences signal des protéines suivies de celles de leurs fibrinopeptides (A et B, respectivement).

Chaque chaîne α ou β de fibrine a été segmentée en séquences contiguës de 15 acides aminés, et tous les peptides comprenant au moins un résidu arginyl ont été choisis. Dans le cas des peptides pour lesquels le résidu arginyl se situait à l'extrémité NH2- ou COOH-terminale, une seconde série de peptides de 15 acides aminés, chevauchant les premiers, de façon à recentrer le résidu arginyl terminal dans la séquence, a été choisie. En outre, un peptide correspondant aux résidus 621-629 de la chaîne α de la fibrine a été synthétisé. Au total, 72 peptides dont 41 dérivés de la chaîne α de la fibrine et 31 dérivés de sa chaîne β ont été choisis. Pour chacun des peptides, la forme avec résidu(s) arginyl (forme native) et la forme où tous les résidus arginyl ont été substitués par des résidus citrullyl (forme citrullinée) ont été synthétisées selon la méthode en phase solide de Merrifield avec une pureté ≥ 60% [Société NeoMPS (Strasbourg, France)].

La liste des peptides citrullinés choisis est donnée dans le Tableau III ci-après :

**Tableau III**

| A. Première série : | | | |
|---|---|---|---|
| Chaîne α | | | |
| α36-50Cit_{38,42} | α171-185Cit_{178,181} | α351-365Cit₃₅₃ | α456-470Cit_{458,459} |
| α66-80Cit₆₉ | α186-200Cit_{186,190} | α366-380Cit₃₆₇ | α501-515Cit_{510,512} |
| α81-95Cit₈₄ | α216-230Cit_{216,218} | α381-395Cit₃₉₄ | α546-560Cit₅₄₇ |
| α111-125Cit_{114,123} | α246-260Cit₂₅₈ | α396-410Cit₄₀₄ | α561-575Cit₅₇₃ |
| α126-140Cit_{129,135,137} | α261-275Cit_{263,271} | α411-425Cit₄₂₅ | α591-605Cit₅₉₁ |
| α141-155Cit₁₄₃ | α276-290Cit₂₈₇ | α426-440Cit₄₂₆ | α621-629Cit_{621,627} |
| α156-170Cit_{160,168} | α306-320Cit₃₀₈ | α441-455Cit₄₄₃ | α621-635Cit_{621,627,630} |

| Chaîne β | | | |
|---|---|---|---|
| β45-59Cit_{47,53} | β195-209Cit_{196,199,206} | β330-344Cit₃₃₄ | β435-449Cit_{436,445} |
| β60-74Cit_{60,72,74}^{a} | β210-224Cit₂₂₄ | β375-389Cit₃₇₆ | β465-479Cit₄₇₈ |
| β75-89Cit₈₇ | β240-254Cit₂₄₆ | β390-404Cit₃₉₅ | β480-491Cit₄₈₅ |
| β120-134Cit_{121,124} | β255-269Cit₂₆₇ | β405-419Cit₄₁₀ | |
| β150-164Cit₁₅₈ | β285-299Cit_{285,294} | β420-434Cit₄₂₁ | |

| B. Deuxième série : | | | |
|---|---|---|---|
| Chaîne α | | | |
| α138-152Cit₁₄₃ | α300-314Cit₃₀₈ | α438-452Cit₄₄₃ | α615-629Cit_{621,627} |
| α183-197Cit_{186,190} | α347-361Cit₃₅₃ | α455-469Cit_{458,459} | |
| α213-227Cit_{216,218} | α363-377Cit₃₆₇ | α542-556Cit₅₄₇ | |
| α259-273Cit_{263,271} | α420-434Cit_{425,426} | α588-602Cit₅₉₁ | |

| Chaîne β | | | |
|---|---|---|---|
| β50-64Cit_{53,60} | β202-216Cit₂₀₆ | β281-295Cit_{285,294} | β474-488Cit_{478,485} |
| β116-130Cit_{121,124} | β215-229Cit₂₂₄ | β373-387Cit₃₇₆ | |
| β188-202Cit_{196,199} | β219-233Cit₂₂₄ | β416-430Cit₄₂₁ | |
| β193-207Cit_{196,199,206} | β236-250Cit₂₄₆ | β433-447Cit_{436,445} | |
| ^{a}Ce peptide a été synthétisé avec la fonction carboxyle (COOH) du résidu citrullyl C-terminal soit sous forme « libre » (COOH), soit sous forme amidée (fonction carboxamide : CONH₂). | | | |

La nomenclature utilisée est la suivante : nom d'origine de la chaîne polypeptidique (α ou β) du fibrinogène dont dérive la séquence, puis position dans cette séquence du résidu amino-terminal du peptide - position du résidu carboxy-terminal du peptide. Ces positions sont numérotées par rapport à l'extrémité N-terminale du fibrinogène. La mention Cit indique qu'il s'agit d'une forme citrullinée du peptide. La position du résidu arginyl qui est substitué par un résidu citrullyl est indiquée en indice. Seules les formes citrullinées des peptides sont présentées.

Chaque paire de peptides (citrulliné et non-citrulliné) a été testée en ELISA avec un mélange à parts égales de 10 sérums ne présentant pas d'AAPC (mélange contrôle) et avec les 2 mélanges A et B décrits dans l'Exemple 1.

Les peptides ont été testés après.revêtement de plaques en polystyrène irradié (Nunc Maxisorp) dans trois tampons différents (acétate pH 5,0, PBS pH 7,4 et carbonate pH 9,0), de manière à optimiser les chances de fixation passive des peptides (10 µg/ml) qui présentaient des points isoélectriques très hétérogènes (s'étalant de 4 à 12 pour les formes non citrullinées). Chaque paire de peptides (forme native et citrullinée) a été testée sur la même plaque et une paire de peptides contrôle - peptide "filaggrine" citrulliné cfc6 et son correspondant natif cf0 (Schellekens GA, 1998, précité) - a été incluse lors de chaque expérimentation, ce qui a permis de calculer un coefficient de variation inter-essais et d'effectuer des corrections.

Après saturation en PBS-BSA 2%, les mélanges de sérums dilués au 1/50 en PBS 2M NaCl - BSA 2% ont été incubés puis leur fixation a été révélée avec des IgG de chèvre anti-IgG humaines marquées à la peroxydase (Southern) diluées au 1/1000 en PBS BSA 2%. Toutes les incubations ont eu lieu pendant 1 h à 4°C et ont été suivies de lavages en PBS-Tween 0,1%. L'activité de la peroxydase a été révélée par une solution d'orthophénylènediamine (2mg/ml - Sigma) en peroxyde d'hydrogène (0,03% - Sigma). La réaction a été stoppée après 5 minutes par de l'acide sulfurique 4M et la densité optique (DO) à 492 nm a été mesurée grâce à un spectrophotomètre automatique (Multiskan, Thermo Labsystems).

La réactivité spécifique des mélanges de sérums à l'égard des peptides citrullinés correspondait à la différence entre la DO obtenue avec le peptide.citrulliné et celle obtenue avec le peptide natif correspondant (delta DO). Les résultats correspondent à la moyenne de 2 déterminations. Tout peptide citrulliné permettant d'obtenir une delta DO supérieure à 0,250 pour au moins un des deux mélanges A et B après revêtement dans au moins un des trois tampons, a été considéré comme réactif.

Parmi les 72 peptides citrullinés analysés, 13 peptides dérivés de la séquence de la chaîne α de la fibrine et 5 peptides dérivés de celle de la chaîne β se sont révélés porteurs d'épitopes reconnus par les AAPC. Parmi ces peptides, 6 peptides étaient très réactifs (delta DO≥1,5), 8 peptides l'étaient moyennement (0,5≤delta DO<1,5) et 4 peptides l'étaient peu (0,25≤delta DO<0,5). Les autres peptides citrullinés se sont avérés peu ou pas réactifs (0,0≤delta DO<0,25). Aucune réactivité avec le mélange contrôle n'a été observée, ce qui permet d'identifier les peptides réactifs comme porteurs d'épitope(s) reconnu(s) par les AAPC.

Les résultats obtenus pour les 18 peptides réactifs sont présentés dans le Tableau IV ci-après :

**Tableau IV**

| Peptide | Mélange de sérums | Tampon de revêtement | | |
|---|---|---|---|---|
| | | Acétate | PBS | Carbonate |
| α36-50Cit_{38,42} | Mélange A | 4,00 | 2,69 | 3,43 |
| | Mélange B | 4,27 | 2,65 | 2,64 |
| α171-185Cit_{178,181} | Mélange A | 0,15 | 0,29 | 0,75 |
| | Mélange B | 0,08 | 0,21 | 0,47 |
| α183-197Cit_{186,190} | Mélange A | 0,41 | 1,68 | 1,36 |
| | Mélange B | 0,01 | 0,15 | 0,07 |
| α246-260Cit₂₅₈ | Mélange A | 0,04 | 0,00 | 0,00 |
| | Mélange B | 0,27 | 0,25 | 0,33 |
| α259-273Cit_{263,271} | Mélange A | 0,14 | 0,60 | 0,12 |
| | Mélange B | 0,21 | 0,69 | 0,20 |
| α366-380Cit₃₆₇ | Mélange A | 0,28 | 0,37 | 0,26 |
| | Mélange B | 0,00 | 0,04 | 0,08 |
| α396-410Cit₄₀₄ | Mélange A | 0,04 | 0,02 | 0,43 |
| | Mélange B | 0,15 | 0,17 | 0,20 |
| α411-425Cit₄₂₅ | Mélange A | 0,09 | 0,16 | 0,56 |
| | Mélange B | 0,38 | 0,66 | 0,43 |
| α501-515Cit_{510,512} | Mélange A | 0,16 | 0,92 | 0,15 |
| | Mélange B | 0,72 | 2,60 | 0,79 |
| α546-560Cit₅₄₇ | Mélange A | 0,38 | 0,74 | 0,33 |
| | Mélange B | 0,05 | 0,15 | 0,16 |
| α561-575Cit₅₇₃ | Mélange A | 0,05 | 0,30 | 0,01 |
| | Mélange B | 0,14 | 0,52 | 0,17 |
| α588-602Cit₅₉₁ | Mélange A | 0,05 | 0,16 | 0,36 |
| | Mélange B | 0,06 | 0,33 | 0,67 |
| α621-635Cit_{621,627,630} | Mélange A | 0,25 | 1,57 | 1,17 |
| | Mélange B | 0,18 | 1,51 | 1,20 |
| β60-74Cit_{60,72,74}^{a} | Mélange A | 1,02 | 2,06 | 1,27 |
| | Mélange B | 2,83 | 2,69 | 2,72 |
| β210-224Cit₂₂₄ | Mélange A | 0,25 | 0,29 | 1,14 |
| | Mélange B | 0,00 | 0,60 | 1,56 |
| β281-295Cit_{285,294} | Mélange A | 0,12 | 0,60 | 0,61 |
| | Mélange B | 0,11 | 0,75 | 0,71 |
| β420-434Cit₄₂₁ | Mélange A | 0,36 | 0,48 | 0,44 |
| | Mélange B | 0,00 | 0,00 | 0,00 |
| β433-447Cit_{436,445} | Mélange A | 0,41 | 0,58 | 0,67 |
| | Mélange B | 0,00 | 0,00 | 0,03 |
| ^{a} Les résultats indiqués correspondent à ceux obtenus avec la forme du peptide ayant une fonction C-terminale amidée (fonction carboxamide : CONH₂) | | | | |

### EXEMPLE 3 : PROFIL DE REACTIVITE DES PEPTIDES CITRULLINES REAGISSANT AVEC LES AAPC.

Les 14 peptides porteurs d'épitopes les plus réactifs (delta DO≥0,5 pour l'un ou l'autre des mélanges de sérum (A et B) avec au moins l' un des trois tampons de revêtement) ont été testés indépendamment avec les 20 sérums constitutifs des mélanges A et B, afin d'évaluer le profil de réactivité de chacun de ces peptides. Les tests ont été effectués en ELISA dans les mêmes conditions que dans l'Exemple 2 ci-dessus, à ceci près que, pour chaque paire de peptides, on a choisi comme tampon de revêtement celui ayant permis d'obtenir la plus forte réactivité vis-à-vis de ce peptide lors du criblage (le tampon où la somme des delta DO obtenues respectivement pour les mélanges A et B de sérums était maximale). En outre, les dilutions des sérums ont été ajustées de telle sorte qu'ils aient une avidité équivalente pour le fibrinogène désiminé entier. Ainsi pour chaque sérum, on a choisi la dilution permettant d'obtenir une DO de 1 en ELISA sur le fibrinogène désiminé (Nogueira L, 2002, précité). Les dilutions s'échelonnaient du 1/20 au 1/2700.

Les résultats sont illustrés par le Tableau V ci-après.

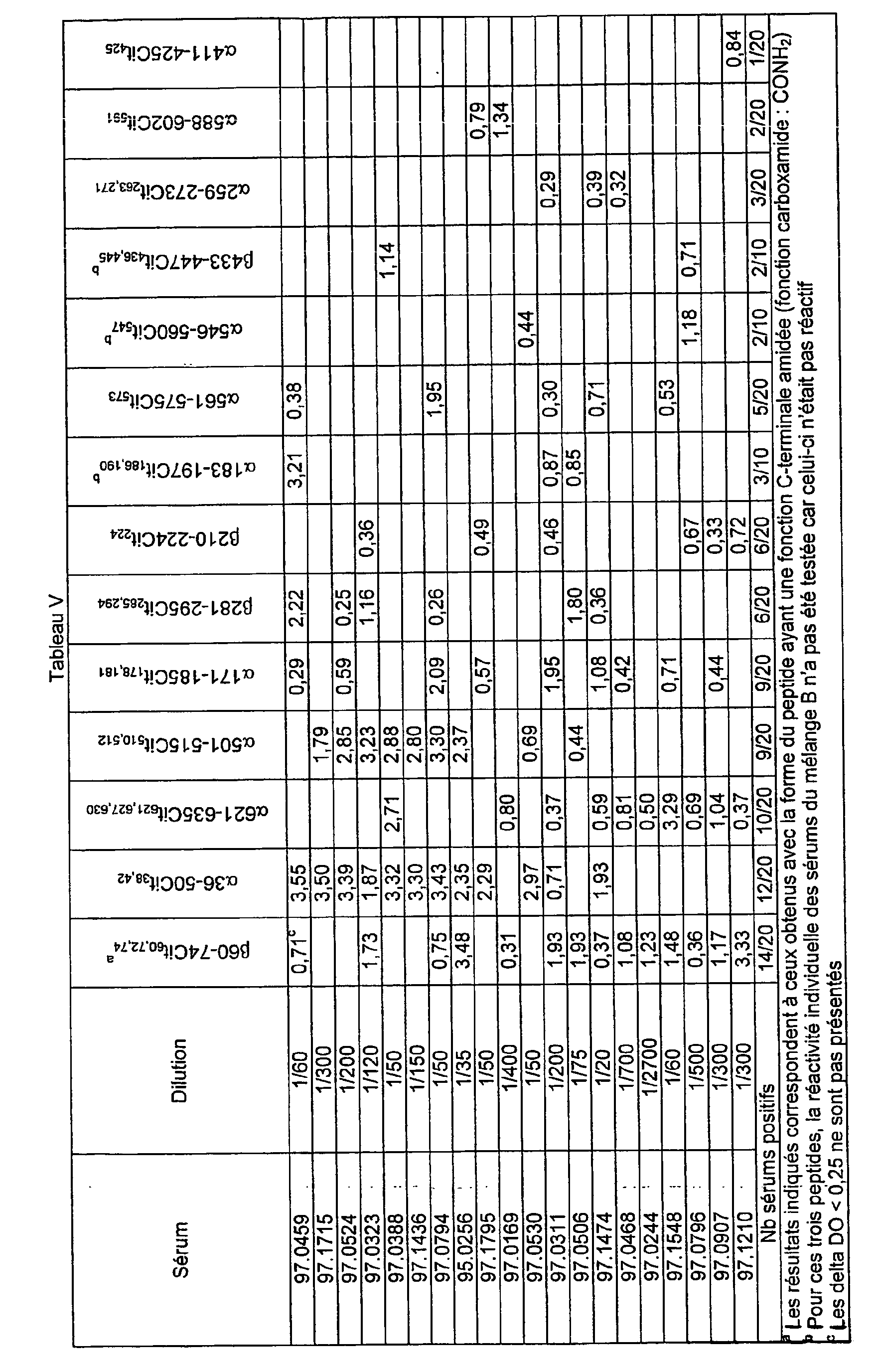

### EXEMPLE 4 : COMPARAISON DE LA REACTIVITE DU PEPTIDE β60-74Cit_{60,72,74} SELON QUE SA FONCTION C-TERMINALE EST UN CARBOXYLE (COOH) OU UN CARBOXAMIDE (CONH₂)

Des plaques ELISA ont été revêtues avec le peptide β60-74Cit_{60,72,74} dont la fonction C-terminale du résidu citrullyl carboxy-terminal n'est pas amidée (forme portant une fonction COOH terminale, ci-après désignée « forme non amidée »), avec le peptide β60-74Cit_{60,72,74} dont la fonction C-terminale du résidu citrullyl carboxy-terminal est amidée (forme portant une fonction CONH₂ terminale, ci-après désignée « forme amidée ») et le peptide non citrulliné β60-74 (dont la fonction C-terminale du résidu arginyl carboxy-terminal n'est pas amidée), tous dilués en PBS. Les mélanges A et B de sérums décrits dans l'exemple 1, ont été testés selon la méthode décrite dans l'exemple 2. Comme dans l'exemple 2, la réactivité spécifique du peptide sous forme non amidée ou sous forme amidée correspondait à la différence entre la DO obtenue avec ces deux peptides citrullinés et celle obtenue avec le peptide non citrulliné β60-74. La réactivité de la forme amidée du peptide est apparue nettement plus forte que celle de la forme non amidée qui était cependant significative. Les résultats correspondent à la moyenne de deux expériences, chacune comprenant deux déterminations. Les résultats sont présentés dans le Tableau VI ci-après.

**Tableau VI**

| Peptide | Mélange de sérums | Delta DO |
|---|---|---|
| β60-74Cit_{60,72,74} non amidé | Mélange A | 0,30 |
| | Mélange B | 1,16 |
| β60-74Cit_{60,72,74} amidé | Mélange A | 2,13 |
| | Mélange B | 2,74 |

## Revendications

1. Peptide isolé reconnu par des auto-anticorps anti-protéines citrullinées (AAPC) présents dans le sérum des patients atteints de polyarthrite rhumatoïde (PR), **caractérisé en ce qu'**il comprend au moins un résidu citrullyl, et **en ce qu'**il est choisi dans le groupe constitué par :
a) un peptide défini par la séquence X₁PAPPPISGGGYX₂AX₃ (SEQ ID NO: 1) dans laquelle X₁, X₂, et X₃ représentent chacun un résidu citrullyl ou un résidu arginyl, et au moins l'un des résidus X₂ ou X₃ est un résidu citrullyl ;
b) un peptide défini par la séquence GPX₁VVEX₂HQSACKDS (SEQ ID NO: 2) dans laquelle X₁ et X₂ représentent chacun un résidu citrullyl ou un résidu arginyl, et au moins l'un des résidus X₁ ou X₂ est un résidu citrullyl ;
c) un peptide défini par la séquence SGIGTLDGFX₁HX₂HPD (SEQ ID NO: 3) dans laquelle X₁ et X₂ représentent chacun un résidu citrullyl ou un résidu arginyl, et au moins l'un des résidus X₁ ou X₂ est un résidu citrullyl ;
d) un peptide défini par la séquence VDIDIKIX₁SCX₂GSCS (SEQ ID NO: 4) dans laquelle X₁ et X₂ représentent chacun un résidu citrullyl ou un résidu arginyl, et au moins l'un des résidus X₁ ou X₂ est un résidu citrullyl ;
e) un peptide défini par la séquence X₁GHAKSX₂PVX₃GIHTS (SEQ ID NO: 12) dans laquelle X₁, X₂ et X₃ représentent chacun un résidu citrullyl ou un résidu arginyl, et au moins l'un des résidus X₁, X₂ ou X₃ est un résidu citrullyl ;
f) un peptide comprenant au moins 5 acides aminés consécutifs, dont au moins un résidu citrullyl, de l'un des peptides a) à e) ci-dessus:

2. Peptide selon la revendication 1,
**caractérisé en ce qu'**il est choisi dans le groupe constitué par :
- un peptide défini par la séquence SEQ ID NO: 1 dans laquelle au moins X₃ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 2 dans laquelle au moins X₂ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 3 dans laquelle au moins X₂ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 4 dans laquelle au moins X₁ est un résidu citrullyl, ou un peptide comprenant un fragment d'au moins 5 acides aminés consécutifs de ladite séquence contenant ledit résidu citrullyl ;
- un peptide défini par la séquence SEQ ID NO: 12 dans laquelle au moins X₃ est un résidu citrullyl, ou un peptide comprenant un fragment d'au moins 5 acides aminés consécutifs de ladite séquence contenant ledit résidu citrullyl.

3. Peptide selon la revendication 2,
**caractérisé en ce qu'**il est choisi dans le groupe constitué par :
- un peptide défini par la séquence SEQ ID NO: 1 dans laquelle X₁, X₂, et X₃ sont des résidus citrullyl, ou un peptide d'au moins 16 acides aminés comprenant ladite séquence ;
- un peptide défini par la séquence SEQ ID NO: 2 dans laquelle X₁ et X₂ sont des résidus citrullyl, ou un peptide comprenant un fragment d'au moins 5 acides aminés consécutifs de ladite séquence contenant lesdits résidus citrullyl ;
- un peptide défini par la séquence SEQ ID NO: 3 dans laquelle X₁ et X₂ sont des résidus citrullyl, ou un peptide comprenant un fragment d'au moins 5 acides aminés consécutifs de ladite séquence contenant lesdits résidus citrullyl ;
- un peptide défini par la séquence SEQ ID NO: 4 dans laquelle X₁ et X₂ sont des résidus citrullyl, ou un peptide comprenant un fragment d'au moins 5 acides aminés consécutifs de ladite séquence contenant lesdits résidus citrullyl ;
- un peptide défini par la séquence SEQ ID NO: 12 dans laquelle X₁, X₂ et X₃ sont des résidus citrullyl, ou un peptide comprenant un fragment d'au moins 10 acides aminés consécutifs de ladite séquence contenant lesdits résidus citrullyl.

4. Peptide selon une quelconque des revendications 1 à 3, dans lequel la fonction carboxyle (COOH) terminale est remplacée par une fonction carboxamide (CONH₂).

5. Utilisation d'un peptide selon une quelconque des revendications 1 à 4 pour détecter la présence d'AAPC spécifiques de la PR dans un échantillon biologique.

6. Composition antigénique, **caractérisée en ce qu'**elle comprend au moins un peptide selon une quelconque des revendications 1 à 4.

7. Composition antigénique selon la revendication 6, **caractérisée en ce qu'**elle comprend un peptide de séquence SEQ ID NO: 1, et un peptide de séquence SEQ ID NO: 2 selon une quelconque des revendications 1 à 4.

8. Composition antigénique selon une quelconque des revendications 6 ou 7, **caractérisée en ce qu'**elle comprend un peptide de séquence SEQ ID NO: 3 et/ou un peptide de séquence SEQ ID NO: 4 et/ou un peptide de séquence SEQ ID NO: 12, selon une quelconque des revendications 1 à 4.

9. Procédé de détection de la présence d'AAPC dans un échantillon biologique lequel procédé est **caractérisé en ce qu'**il comprend :
- la mise en contact dudit échantillon biologique avec au moins un peptide selon une quelconque des revendications 1 à 4 ou une composition antigénique selon une quelconque des revendications 6 à 8, dans des conditions permettant la formation d'un complexe antigène/anticorps avec les AAPC éventuellement présents dans ledit échantillon ;
- la détection, par tous moyens appropriés, du complexe antigène/anticorps éventuellement formé.

10. Procédé de détection de la présence d'AAPC dans un échantillon biologique lequel procédé est **caractérisé en ce qu'**il comprend :
- la fourniture d'un premier peptide citrulliné capable d'entrer en compétition pour la liaison auxdits AAPC avec un peptide tel que défini dans une quelconque des revendications 1 à 4 ;
- la mise en contact dudit premier peptide avec ledit échantillon biologique dans des conditions permettant la formation d'un complexe antigène/anticorps avec les AAPC éventuellement présents dans ledit échantillon ;
- la détection, par tous moyens appropriés, du complexe antigène/anticorps éventuellement formé.

## Claims

1. Isolated peptide recognised by anti-citrullinated protein auto-antibodies (ACPAs) present in the serum of patients suffering from rheumatoid polyarthritis (RP), **characterised in that** it comprises at least one citrullyl residue and **in that** it is selected from the group constituted by:
a) a peptide defined by the sequence X₁PAPPPISGGGYX₂AX₃ (SEQ ID NO: 1) in which X₁, X₂ and X₃ each represents a citrullyl residue or an arginyl residue and at least one of the residues X₂ or X₃ is a citrullyl residue;
b) a peptide defined by the sequence GPX₁VVEX₂HQSACKDS (SEQ ID NO: 2) in which X₁ and X₂ each represents a citrullyl residue or an arginyl residue and at least one of the residues X₁ or X₂ is a citrullyl residue;
c) a peptide defined by the sequence SGIGTLDGFX₁HX₂HPD (SEQ ID NO: 3) in which X₁ and X₂ each represents a citrullyl residue or an arginyl residue and at least one of the residues X₁ or X₂ is a citrullyl residue;
d) a peptide defined by the sequence VDIDIKIX₁SCX₂GSCS (SEQ ID NO: 4) in which X₁ and X₂ each represents a citrullyl residue or an arginyl residue and at least one of the residues X₁ or X₂ is a citrullyl residue;
e) a peptide defined by the sequence X₁GHAKSX₂PVX₃GIHTS (SEQ ID NO: 12) in which X₁, X₂ and X₃ each represents a citrullyl residue or an arginyl residue and at least one of the residues X₁, X₂ or X₃ is a citrullyl residue;
f) a peptide comprising at least 5 consecutive amino acids, including at least one citrullyl residue, of one of peptides a) to e) above.

2. Peptide according to claim 1, **characterised in that** it is selected from the group constituted by:
- a peptide defined by sequence SEQ ID NO: 1 in which at least X₃ is a citrullyl residue, or a peptide comprising a fragment of at least 5 consecutive amino acids of said sequence containing said citrullyl residue;
- a peptide defined by sequence SEQ ID NO: 2 in which at least X₂ is a citrullyl residue, or a peptide comprising a fragment of at least 5 consecutive amino acids of said sequence containing said citrullyl residue;
- a peptide defined by sequence SEQ ID NO: 3 in which at least X₂ is a citrullyl residue, or a peptide comprising a fragment of at least 5 consecutive amino acids of said sequence containing said citrullyl residue;
- a peptide defined by sequence SEQ ID NO: 4 in which at least X₁ is a citrullyl residue, or a peptide comprising a fragment of at least 5 consecutive amino acids of said sequence containing said citrullyl residue;
- a peptide defined by sequence SEQ ID NO: 12 in which at least X₃ is a citrullyl residue, or a peptide comprising a fragment of at least 5 consecutive amino acids of said sequence containing said citrullyl residue.

3. Peptide according to claim 2, **characterised in that** it is selected from the group constituted by:
- a peptide defined by sequence SEQ ID NO: 1 in which X₁, X₂ and X₃ are citrullyl residues, or a peptide of at least 16 amino acids comprising said sequence;
- a peptide defined by sequence SEQ ID NO: 2 in which X₁ and X₂ are citrullyl residues, or a peptide comprising a fragment of at least 5 consecutive amino acids of said sequence containing said citrullyl residues;
- a peptide defined by sequence SEQ ID NO: 3 in which X₁ and X₂ are citrullyl residues, or a peptide comprising a fragment of at least 5 consecutive amino acids of said sequence containing said citrullyl residues;
- a peptide defined by sequence SEQ ID NO: 4 in which X₁ and X₂ are citrullyl residues, or a peptide comprising a fragment of at least 5 consecutive amino acids of said sequence containing said citrullyl residues;
- a peptide defined by sequence SEQ ID NO: 12 in which X₁, X₂ and X₃ are citrullyl residues, or a peptide comprising a fragment of at least 10 consecutive amino acids of said sequence containing said citrullyl residues.

4. Peptide according to any one of claims 1 to 3, in which the terminal carboxyl (COOH) function is replaced by a carboxamide (CONH₂) function.

5. Use of a peptide according to any one of claims 1 to 4 for detecting the presence of RP-specific ACPAs in a biological sample.

6. Antigenic composition, **characterised in that** it comprises at least one peptide according to any one of claims 1 to 4.

7. Antigenic composition according to claim 6, **characterised in that** it comprises a peptide of sequence SEQ ID NO: 1 and a peptide of sequence SEQ ID NO: 2 according to any one of claims 1 to 4.

8. Antigenic composition according to either claim 6 or claim 7, **characterised in that** it comprises a peptide of sequence SEQ ID NO: 3 and/or a peptide of sequence SEQ ID NO: 4 and/or a peptide of sequence SEQ ID NO: 12 according to any one of claims 1 to 4.

9. Method for detecting the presence of ACPAs in a biological sample, which method is **characterised in that** it comprises:
- bringing said biological sample into contact with at least one peptide according to any one of claims 1 to 4 or with an antigenic composition according to any one of claims 6 to 8, under conditions permitting the formation of an antigen/antibody complex with the ACPAs optionally present in said sample;
- detecting, by any suitable means, the antigen/antibody complex optionally formed.

10. Method for detecting the presence of ACPAs in a biological sample, which method is **characterised in that** it comprises:
- providing a first citrullinated peptide capable of competing, for binding to said ACPAs, with a peptide as defined in any one of claims 1 to 4;
- bringing said first peptide into contact with said biological sample under conditions permitting the formation of an antigen/antibody complex with the ACPAs optionally present in said sample;
- detecting, by any suitable means, the antigen/antibody complex optionally formed.

## Patentansprüche

1. Isoliertes Peptid, das von Autoantikörpern gegen citrullinierte Proteine (AAPC) erkannt wird, die sich im Serum von Patienten befinden, die an rheumatoider Polyarthritis (PR) leiden, **dadurch gekennzeichnet, dass** es wenigstens einen Citrullylrest umfasst, und **dadurch**, dass es ausgewählt ist aus der Gruppe bestehend aus:
a) einem durch die Sequenz X₁ PAPPPISGGGYX₂AX₃ (SEQ ID NO:1) definierten Peptid, in dem X₁, X₂ und X₃ jeweils einen Citrullylrest oder einen Arginylrest darstellen und wenigstens einer der Reste X₂ oder X₃ ein Citrullylrest ist;
b) einem durch die Sequenz GPX₁VVEX₂HQSACKDS (SEQ ID NO:2) definierten Peptid, in dem X₁ und X₂ jeweils einen Citrullylrest oder einen Arginylrest darstellen und wenigstens einer der Reste X₁ oder X₂ ein Citrullylrest ist;
c) einem durch die Sequenz SGIGTLDGFX₁HX₂HPD (SEQ ID NO:3) definierten Peptid, in dem X₁ und X₂ jeweils einen Citrullylrest oder einen Arginylrest darstellen und wenigstens einer der Reste X₁ oder X₂ ein Citrullylrest ist;
d) einem durch die Sequenz VDIDIKIX₁SCX₂GSCS (SEQ ID NO:4) definierten Peptid, in dem X₁ und X₂ jeweils einen Citrullylrest oder einen Arginylrest darstellen und wenigstens einer der Reste X₁ oder X₂ ein Citrullylrest ist;
e) einem durch die Sequenz X₁GHAKSX₂PVX₃GIHTS (SEQ ID NO:12) definierten Peptid, in dem X₁, X₂ und X₃ jeweils einen Citrullylrest oder einen Arginylrest darstellen und wenigstens einer der Reste X₁, X₂ oder X₃ ein Citrullylrest ist;
f) einem Peptid, das wenigstens 5 aufeinanderfolgende Aminosäuren, darunter wenigstens einen Citrullylrest, von einem der obigen Peptide a) bis e) umfasst.

2. Peptid nach Anspruch 1, **dadurch gekennzeichnet, dass** es ausgewählt ist aus der Gruppe bestehend aus:
- einem durch die Sequenz SEQ ID NO:1 definierten Peptid, in dem wenigstens X₃ ein Citrullylrest ist, oder einem Peptid, das ein Fragment mit wenigstens 5 aufeinanderfolgenden Aminosäuren dieser Sequenz umfasst, die diesen Citrullylrest enthalten;
- einem durch die Sequenz SEQ ID NO:2 definierten Peptid, in dem wenigstens X₂ ein Citrullylrest ist, oder einem Peptid, das ein Fragment mit wenigstens 5 aufeinanderfolgenden Aminosäuren dieser Sequenz umfasst, die diesen Citrullylrest enthalten;
- einem durch die Sequenz SEQ ID NO:3 definierten Peptid, in dem wenigstens X₂ ein Citrullylrest ist, oder einem Peptid, das ein Fragment mit wenigstens 5 aufeinanderfolgenden Aminosäuren dieser Sequenz umfasst, die diesen Citrullylrest enthalten;
- einem durch die Sequenz SEQ ID NO:4 definierten Peptid, in dem wenigstens X₁ ein Citrullylrest ist, oder einem Peptid, das ein Fragment mit wenigstens 5 aufeinanderfolgenden Aminosäuren dieser Sequenz umfasst, die diesen Citrullylrest enthalten;
- einem durch die Sequenz SEQ ID NO:12 definierten Peptid, in dem wenigstens X₃ ein Citrullylrest ist, oder einem Peptid, das ein Fragment mit wenigstens 5 aufeinanderfolgenden Aminosäuren dieser Sequenz umfasst, die diesen Citrullylrest enthalten.

3. Peptid nach Anspruch 2, **dadurch gekennzeichnet, dass** es ausgewählt ist aus der Gruppe bestehend aus:
- einem durch die Sequenz SEQ ID NO:1 definierten Peptid, in dem X₁, X₂ und X₃ Citrullylreste sind, oder einem Peptid mit wenigstens 16 Aminosäuren, das diese Sequenz umfasst;
- einem durch die Sequenz SEQ ID NO:2 definierten Peptid, in dem X₁ und X₂ Citrullylreste sind, oder einem Peptid, das ein Fragment mit wenigstens 5 aufeinanderfolgenden Aminosäuren dieser Sequenz umfasst, die diese Citrullylreste enthalten;
- einem durch die Sequenz SEQ ID NO:3 definierten Peptid, in dem X₁ und X₂ Citrullylreste sind, oder einem Peptid, das ein Fragment mit wenigstens 5 aufeinanderfolgenden Aminosäuren dieser Sequenz umfasst, die diese Citrullylreste enthalten;
- einem durch die Sequenz SEQ ID NO:4 definierten Peptid, in dem X₁ und X₂ Citrullylreste sind, oder einem Peptid, das ein Fragment mit wenigstens 5 aufeinanderfolgenden Aminosäuren dieser Sequenz umfasst, die diese Citrullylreste enthalten;
- einem durch die Sequenz SEQ ID NO:12 definierten Peptid, in dem X₁, X₂ und X₃ Citrullylreste sind, oder einem Peptid, das ein Fragment mit wenigstens 10 aufeinanderfolgenden Aminosäuren dieser Sequenz umfasst, die diese Citrullylreste enthalten.

4. Peptid nach einem der Ansprüche 1 bis 3, in dem die terminale Carboxylfunktion (COOH) durch eine Carboxamidfunktion (CONH₂) ersetzt ist.

5. Verwendung eines Peptids nach einem der Ansprüche 1 bis 4 zum Nachweis der Anwesenheit von für PR spezifischen AAPC in einer biologischen Probe.

6. Antigene Zusammensetzung, **dadurch gekennzeichnet, dass** sie wenigstens ein Peptid nach einem der Ansprüche 1 bis 4 umfasst.

7. Antigene Zusammensetzung nach Anspruch 6, **dadurch gekennzeichnet, dass** sie ein Peptid der Sequenz SEQ ID NO:1 und ein Peptid der Sequenz SEQ ID NO:2 nach einem der Ansprüche 1 bis 4 umfasst.

8. Antigene Zusammensetzung nach einem der Ansprüche 6 oder 7, **dadurch gekennzeichnet, dass** sie ein Peptid der Sequenz SEQ ID NO:3 und/oder ein Peptid der Sequenz SEQ ID NO:4 und/oder ein Peptid der Sequenz SEQ ID NO:12 nach einem der Ansprüche 1 bis 4 umfasst.

9. Verfahren zum Nachweis der Anwesenheit von AAPC in einer biologischen Probe, wobei das Verfahren **dadurch gekennzeichnet ist, dass** es umfasst:
- Inkontaktbringen der biologischen Probe mit wenigstens einem Peptid nach einem der Ansprüche 1 bis 4 oder einer antigenen Zusammensetzung nach einem der Ansprüche 6 bis 8 unter Bedingungen, die die Bildung eines Antigen/Antikörper-Komplexes mit den gegebenenfalls in der Probe befindlichen AAPC erlauben;
- Nachweis, mit allen geeigneten Mitteln, des gegebenenfalls gebildeten Antigen/Antikörper-Komplexes.

10. Verfahren zum Nachweis der Anwesenheit von AAPC in einer biologischen Probe, wobei das Verfahren **dadurch gekennzeichnet ist, dass** es umfasst:
- Bereitstellen eines ersten citrullinierten Peptids, das mit einem Peptid, wie es in einem der Ansprüche 1 bis 4 definiert ist, um die Bindung an diese AAPC konkurrieren kann;
- Inkontaktbringen des ersten Peptids mit der biologischen Probe unter Bedingungen, die die Bildung eines Antigen/Antikörper-Komplexes mit den gegebenenfalls in der Probe befindlichen AAPC erlauben;
- Nachweis, mit allen geeigneten Mitteln, des gegebenenfalls gebildeten Antigen/Antikörper-Komplexes.


### SEQUENCE LISTING

<110> BIOMERIEUX
   SERRE, Guy
   SEBBAG, Mireille
<120> Peptides citrullinés dérivés de la fibrine reconnus par des auto-anticorps spécifiques de la polyarthrite rhumatoïde,
   et leurs utilisations.
<130> MJPbv-F1067/2
<150> FR 0411782
   <151> 2004-11-04
<150> FR 0413711
   <151> 2004-12-22
<150> FR 0508422
   <151> 2005-08-08
<160> 12
<170> PatentIn version 3.3
<210> 1
   <211> 15
   <212> PRT
   <213> Artificial Sequence
<220>
   <223> Peptide citrulliné
<220>
   <221> misc_feature
   <222> (1)..(1)
   <223> X = résidu citrullyl ou arginyl
<220>
   <221> misc_feature
   <222> (13) .. (13)
   <223> X = résidu citrullyl ou arginyl
<220>
   <221> misc_feature
   <222> (15)..(15)
   <223> X = résidu citrullyl ou arginyl
<400> 1
   Xaa Pro Ala Pro Pro Pro Ile Ser Gly Gly Gly Tyr Xaa Ala Xaa
   1               5                   10                  15
<210> 2
   <211> 15
   <212> PRT
   <213> Artificial sequence
<220>
   <223> Peptide citrulliné
<220>
   <221> misc_feature
   <222> (3)..(3)
   <223> X = résidu citrullyl ou arginyl
<220>
   <221> misc_feature
   <222> (7) .. (7)
   <223> X = résidu citrullyl ou arginyl
<400> 2
   Gly Pro Xaa Val Val Glu Xaa His Gln Ser Ala Cys Lys Asp Ser
   1               5                   10                  15
<210> 3
   <211> 15
   <212> PRT
   <213> Artificial sequence
<220>
   <223> Peptide citrulliné
<220>
   <221> misc_feature
   <222> (10)..(10)
   <223> X = résidu citrullyl ou arginyl
<220>
   <221> misc_feature
   <222> (12)..(12)
   <223> X = résidu citrullyl ou arginyl
<400> 3
   Ser Gly Ile Gly Thr Leu Asp Gly Phe Xaa His Xaa His Pro Asp
   1               5                   10                  15
<210> 4
   <211> 15
   <212> PRT
   <213> Artificial sequence
<220>
   <223> Peptide citrulliné
<220>
   <221> misc_feature
   <222> (8) .. (8)
   <223> X = résidu citrullyl ou arginyl
<220>
   <221> misc_feature
   <222> (11)..(11)
   <223> X = résidu citrullyl ou arginyl
<400> 4
   Val Asp Ile Asp Ile Lys Ile Xaa Ser Cys Xaa Gly Ser Cys Ser
   1               5                   10                  15
<210> 5
   <211> 14
   <212> PRT
   <213> Artificial sequence
<220>
   <223> Peptide citrulliné
<220>
   <221> misc_feature
   <222> (8)..(8)
   <223> X = résidu citrullyl
<400> 5
   Glu Ser Ser Arg Asp Gly Ser Xaa His Pro Arg Ser His Asp
   1 5 10
<210> 6
   <211> 14
   <212> PRT
   <213> Artificial sequence
<220>
   <223> Peptide citrulliné
<220>
   <221> misc_feature
   <222> (8)..(8)
   <223> X = résidu citrullyl
<400> 6
   Thr Gly Ser Ser Thr Gly Gly Xaa Gln Gly Ser His His Glu
   1 5 10
<210> 7
   <211> 14
   <212> PRT
   <213> Artificial sequence
<220>
   <223> Peptide citrulliné
<220>
   <221> misc_feature
   <222> (8) .. (8)
   <223> X = résidu citrullyl
<400> 7
   Glu Gln Ser Ala Asp Ser Ser Xaa His Ser Gly Ser Gly His
   1 5 10
<210> 8
   <211> 19
   <212> PRT
   <213> Artificial sequence
<220>
   <223> Peptide citrulliné
<220>
   <221> misc_feature
   <222> (7)..(7)
   <223> X = résidu citrullyl
<220>
   <221> misc_feature
   <222> (9) .. (9)
   <223> X = résidu citrullyl
<400> 8
   Ser His Gln Glu Ser Thr Xaa Gly Xaa Ser Arg Gly Arg Ser Gly Arg
   1               5                   10                  15
   Ser Gly Ser
<210> 9
   <211> 18
   <212> PRT
   <213> Artificial sequence
<220>
   <223> Peptide citrulliné
<220>
   <221> misc_feature
   <222> (9)..(10)
   <223> X = résidu citrullyl
<400> 9
   Thr Ile His Ala His Pro Gly Ser Xaa Xaa Gly Gly Arg His Gly Tyr
   1               5                   10                  15
   His His
<210> 10
   <211> 635
   <212> PRT
   <213> Homo sapiens
<400> 10
   Met Phe Ser Met Arg Ile Val Cys Leu Val Leu Ser Val Val Gly Thr
   1               5                   10                  15
   Ala Trp Thr Ala Asp Ser Gly Glu Gly Asp Phe Leu Ala Glu Gly Gly
               20                  25                  30
   Gly Val Arg Gly Pro Arg Val Val Glu Arg His Gln Ser Ala Cys Lys
           35                  40                  45
   Asp Ser Asp Trp Pro Phe Cys Ser Asp Glu Asp Trp Asn Tyr Lys Cys
       50                  55                  60
   Pro Ser Gly Cys Arg Met Lys Gly Leu Ile Asp Glu Val Asn Gln Asp
   65                  70                  75                  80
   Phe Thr Asn Arg Ile Asn Lys Leu Lys Asn Ser Leu Phe Glu Tyr Gln
                   85                  90                  95
   Lys Asn Asn Lys Asp Ser His Ser Leu Thr Thr Asn Ile Met Glu Ile
               100                 105                 110
   Leu Arg Gly Asp Phe Ser Ser Ala Asn Asn Arg Asp Asn Thr Tyr Asn
           115                 120                 125
   Arg Val Ser Glu Asp Leu Arg Ser Arg Ile Glu Val Leu Lys Arg Lys
       130                 135                 140
   Val Ile Glu Lys Val Gln His Ile Gln Leu Leu Gln Lys Asn Val Arg
   145                 150                 155                 160
   Ala Gln Leu Val Asp Met Lys Arg Leu Glu Val Asp Ile Asp Ile Lys
                   165                 170                 175
   Ile Arg Ser Cys Arg Gly Ser Cys Ser Arg Ala Leu Ala Arg Glu Val
               180                 185                 190
   Asp Leu Lys Asp Tyr Glu Asp Gln Gln Lys Gln Leu Glu Gln Val Ile
           195                 200                 205
   Ala Lys Asp Leu Leu Pro Ser Arg Asp Arg Gln His Leu Pro Leu Ile
       210                 215                 220
   Lys Met Lys Pro Val Pro Asp Leu Val Pro Gly Asn Phe Lys Ser Gln
   225                 230                 235                 240
   Leu Gln Lys Val Pro Pro Glu Trp Lys Ala Leu Thr Asp Met Pro Gln
                   245                 250                 255
   Met Arg Met Glu Leu Glu Arg Pro Gly Gly Asn Glu Ile Thr Arg Gly
               260                 265                 270
   Gly Ser Thr Ser Tyr Gly Thr Gly Ser Glu Thr Glu Ser Pro Arg Asn
           275                 280                 285
   Pro Ser Ser Ala Gly Ser Trp Asn Ser Gly Ser Ser Gly Pro Gly Ser
       290                 295                 300
   Thr Gly Asn Arg Asn Pro Gly Ser Ser Gly Thr Gly Gly Thr Ala Thr
   305                 310                 315                 320
   Trp Lys Pro Gly Ser Ser Gly Pro Gly Ser Thr Gly Ser Trp Asn Ser
                   325                 330                 335
   Gly Ser Ser Gly Thr Gly Ser Thr Gly Asn Gln Asn Pro Gly Ser Pro
               340                 345                 350
   Arg Pro Gly Ser Thr Gly Thr Trp Asn Pro Gly Ser Ser Glu Arg Gly
           355                 360                 365
   Ser Ala Gly His Trp Thr Ser Glu Ser Ser Val Ser Gly Ser Thr Gly
       370                 375                 380
   Gln Trp His Ser Glu Ser Gly Ser Phe Arg Pro Asp Ser Pro Gly Ser
   385                 390                 395                 400
   Gly Asn Ala Arg Pro Asn Asn Pro Asp Trp Gly Thr Phe Glu Glu Val
                   405                 410                 415
   Ser Gly Asn Val Ser Pro Gly Thr Arg Arg Glu Tyr His Thr Glu Lys
               420                 425                 430
   Leu Val Thr Ser Lys Gly Asp Lys Glu Leu Arg Thr Gly Lys Glu Lys
           435                 440                 445
   Val Thr Ser Gly Ser Thr Thr Thr Thr Arg Arg Ser Cys Ser Lys Thr
       450                 455                 460
   Val Thr Lys Thr Val Ile Gly Pro Asp Gly His Lys Glu Val Thr Lys
   465                 470                 475                 480
   Glu Val Val Thr Ser Glu Asp Gly Ser Asp Cys Pro Glu Ala Met Asp
                   485                 490                 495
   Leu Gly Thr Leu Ser Gly Ile Gly Thr Leu Asp Gly Phe Arg His Arg
               500                 505                 510
   His Pro Asp Glu Ala Ala Phe Phe Asp Thr Ala Ser Thr Gly Lys Thr
           515                 520                 525
   Phe Pro Gly Phe Phe Ser Pro Met Leu Gly Glu Phe Val Ser Glu Thr
       530                 535                 540
   Glu Ser Arg Gly Ser Glu Ser Gly Ile Phe Thr Asn Thr Lys Glu Ser
   545                 550                 555                 560
   Ser Ser His His Pro Gly Ile Ala Glu Phe Pro Ser Arg Gly Lys Ser
                   565                 570                 575
   Ser Ser Tyr Ser Lys Gln Phe Thr Ser Ser Thr Ser Tyr Asn Arg Gly
               580                 585                 590
   Asp Ser Thr Phe Glu Ser Lys Ser Tyr Lys Met Ala Asp Glu Ala Gly
           595                 600                 605
   Ser Glu Ala Asp His Glu Gly Thr His Ser Thr Lys Arg Gly His Ala
       610                 615                 620
   Lys Ser Arg Pro Val Arg Gly Ile His Thr Ser
   625                 630                 635
<210> 11
   <211> 491
   <212> PRT
   <213> Homo sapiens
<400> 11
   Met Lys Arg Met Val Ser Trp Ser Phe His Lys Leu Lys Thr Met Lys
   1               5                   10                  15
   His Leu Leu Leu Leu Leu Leu Cys Val Phe Leu Val Lys Ser Gln Gly
               20                  25                  30
   Val Asn Asp Asn Glu Glu Gly Phe Phe Ser Ala Arg Gly His Arg Pro
           35                  40                  45
   Leu Asp Lys Lys Arg Glu Glu Ala Pro Ser Leu Arg Pro Ala Pro Pro
       50                  55                  60
   Pro Ile Ser Gly Gly Gly Tyr Arg Ala Arg Pro Ala Lys Ala Ala Ala
   65                  70                  75                  80
   Thr Gln Lys Lys Val Glu Arg Lys Ala Pro Asp Ala Gly Gly Cys Leu
                   85                  90                  95
   His Ala Asp Pro Asp Leu Gly Val Leu Cys Pro Thr Gly Cys Gln Leu
               100                 105                 110
   Gln Glu Ala Leu Leu Gln Gln Glu Arg Pro Ile Arg Asn Ser Val Asp
           115                 120                 125
   Glu Leu Asn Asn Asn Val Glu Ala Val Ser Gln Thr Ser Ser Ser Ser
       130                 135                 140
   Phe Gln Tyr Met Tyr Leu Leu Lys Asp Leu Trp Gln Lys Arg Gln Lys
   145                 150                 155                 160
   Gln Val Lys Asp Asn Glu Asn Val Val Asn Glu Tyr Ser Ser Glu Leu
                   165                 170                 175
   Glu Lys His Gln Leu Tyr Ile Asp Glu Thr Val Asn Ser Asn Ile Ala
               180                 185                 190
   Thr Asn Leu Arg Val Leu Arg Ser Ile Leu Glu Asn Leu Arg Ser Lys
           195                 200                 205
   Ile Gln Lys Leu Glu Ser Asp Val Ser Ala Gln Met Glu Tyr Cys Arg
       210                 215                 220
   Thr Pro Cys Thr Val Ser Cys Asn Ile Pro Val Val Ser Gly Lys Glu
   225                 230                 235                 240
   Cys Glu Glu Ile Ile Arg Lys Gly Gly Glu Thr Ser Glu Met Tyr Leu
                   245                 250                 255
   Ile Gln Pro Asp Ser Ser Val Lys Pro Tyr Arg Val Tyr Cys Asp Met
               260                 265                 270
   Asn Thr Glu Asn Gly Gly Trp Thr Val Ile Gln Asn Arg Gln Asp Gly
           275                 280                 285
   Ser Val Asp Phe Gly Arg Lys Trp Asp Pro Tyr Lys Gln Gly Phe Gly
       290                 295                 300
   Asn Val Ala Thr Asn Thr Asp Gly Lys Asn Tyr Cys Gly Leu Pro Gly
   305                 310                 315                 320
   Glu Tyr Trp Leu Gly Asn Asp Lys Ile Ser Gln Leu Thr Arg Met Gly
                   325                 330                 335
   Pro Thr Glu Leu Leu Ile Glu Met Glu Asp Trp Lys Gly Asp Lys Val
               340                 345                 350
   Lys Ala His Tyr Gly Gly Phe Thr Val Gln Asn Glu Ala Asn Lys Tyr
           355                 360                 365
   Gln Ile Ser Val Asn Lys Tyr Arg Gly Thr Ala Gly Asn Ala Leu Met
       370                 375                 380
   Asp Gly Ala Ser Gln Leu Met Gly Glu Asn Arg Thr Met Thr Ile His
   385 390 395 400
   Asn Gly Met Phe Phe Ser Thr Tyr Asp Arg Asp Asn Asp Gly Trp Leu
   405 410 415
   Thr Ser Asp Pro Arg Lys Gln Cys Ser Lys Glu Asp Gly Gly Gly Trp
   420 425 430
   Trp Tyr Asn Arg Cys His Ala Ala Asn Pro Asn Gly Arg Tyr Tyr Trp
   435 440 445
   Gly Gly Gln Tyr Thr Trp Asp Met Ala Lys His Gly Thr Asp Asp Gly
   450 455 460
   Val Val Trp Met Asn Trp Lys Gly Ser Trp Tyr Ser Met Arg Lys Met
   465 470 475 480
   Ser Met Lys Ile Arg Pro Phe Phe Pro Gln Gln
   485 490
<210> 12
   <211> 15
   <212> PRT
   <213> Artificial Sequence
<220>
   <223> Peptide citrulliné
<220>
   <221> misc_feature
   <222> (1) .. (1)
   <223> X = résidu citrullyl ou arginyl
<220>
   <221> misc_feature
   <222> (7) .. (7)
   <223> X = résidu citrullyl ou arginyl
<220>
   <221> misc_feature
   <222> (10) .. (10)
   <223> X = résidu citrullyl ou arginyl
<400> 12
   Xaa Gly His Ala Lys Ser Xaa Pro Val Xaa Gly Ile His Thr Ser
   1 5 10 15